# EUROPEAN PATENT SPECIFICATION

(11) **EP 2 414 563 B1**
(45) Date of publication and mention of the grant of the patent: **16.03.2016**
(21) Application number: 09776504.4
(22) Date of filing: 02.04.2009
(51) Int. Cl.: C25D 9/04, A61L 27/32, A61L 27/54, A61L 31/08, C25D 9/08, A61L 31/16

(54) **A METHOD OF SURFACE TREATMENT OF AN IMPLANT, AN IMPLANT TREATED BY SAID METHOD AND AN ELECTROLYTE SOLUTION FOR USE IN SAID METHOD**
VERFAHREN ZUR OBERFLÄCHENBEHANDLUNG EINES IMPLANTATS UND NACH DEM VERFAHREN BEHANDELTES IMPLANTAT UND ELEKTROLYTLÖSUNG ZUR VERWENDUNG BEI DEM VERFAHREN
PROCÉDÉ DE TRAITEMENT DE SURFACE D'UN IMPLANT, IMPLANT TRAITÉ PAR LEDIT PROCÉDÉ ET SOLUTION ÉLECTROLYTIQUE DESTINÉE À ÊTRE UTILISÉE DANS LEDIT PROCÉDÉ

(43) Date of publication of application: 08.02.2012
(73) Proprietor: Smith & Nephew Orthopaedics AG, 6343 Rotkreuz (CH)
(72) Inventor: FRISCHHERZ, Marina, CH-6030 Ebikon (CH); FRAUCHIGER, Vinzenz, Max, CH-4500 Solothurn (CH)
(74) Representative: Smith & Nephew
(86) International application number: PCT/EP2009/002431
(87) International publication number: WO 2010/112044

(56) References cited:
- WO-A-98/13539
- WO-A-2007/124572
- WO-A-2008/096160
- US-A1- 2004 033 249
- US-A1- 2005 019 365
- US-A1- 2007 181 221
- US-A1- 2008 011 613

## Description

The present invention relates to a method of surface treatment of an implant, an implant treated in accordance with this method and an electrolyte solution for use in the method.

Implant-related infection following joint replacement is a serious complication in orthopaedic surgery and in extreme cases may require removal of the prosthesis. Infections develop primarily because the interface between artificial implant and tissue is not as well supplied with blood as other parts of the tissue so that parenteral drug administration may not be effective. Antibiotics have to be applied locally to fight infection and to kill the pathogenic germs completely. In the past this has been attempted by bonding antimicrobials directly to the surface of the implant or by incorporating conventional antibiotics into resorbable polymer coatings, allografts, acrylic cements, or calcium phosphate cements. However, these methods tend to impede good osteointegration of the artificial implant surface.

More recently, calcium phosphate implant coatings with antimicrobial properties have been produced by the incorporation of silver ions into the coating. Calcium phosphate coatings, which mostly consist of hydroxyapatite, allow for direct and chemical bone bonding to the artificial implant surface. One method of depositing such coatings onto implant surfaces is a plasma spray technique. However, if silver ions have to be included into the plasma sprayed coatings, this is time consuming and sprayed coatings can only be applied to surfaces of the implant in the line of sight, which is a major disadvantage of this method. Another method is via an electrochemical (i.e. cathodic) deposition of brushite on to the implant but a drawback of electrochemically deposited coatings is their limited mechanical integrity. On the other hand, this method has the advantage that it can be applied to any conductive implant grade substrate. However, in order to incorporate silver ions into such coatings a post treatment has to be applied whereby an ion exchange reaction is produced in a silver nitrate solution. Yet another method is to use sol/gel coatings which are applied to the implant using simple dipping techniques but in order to form a dense gel such coatings have to undergo a heat treatment that may have detrimental effects on the substrate.

US2008/0011613 describes a method of electrolytically depositing a pharmaceutical coating onto a conductive osteal implant.

US2004/0033249 describes a process for electrochemically coating metallic implant materials with a mineralised collagen matrix. US2005/0019365 describes a method for fabricating a bioactive, porous and calcium containing surface layer on medical implants. WO2007/124572 describes a method for electrolytic co-deposition of calcium phosphate and drug composites.

WO2008/096160 describes a method for the electrochemical deposition of a layer containing calcium and phosphorus ions onto a metallic substrate and the incorporation of a therapeutic agent into the electrochemically deposited layer. WO98/13539 describes a method for coating a calcium phosphate compound onto a metallic coating. The method utilises electrodeposition of a phosphate compound from an aqueous solution containing calcium ions, phosphate ions and a complex forming agent onto an electrode comprising the metallic material.

An object of the present invention is to provide a method of surface treatment of an implant and thereby an implant treated by this method which overcomes the aforementioned disadvantages by simultaneously electrochemically depositing a therapeutic agent and a calcium phosphate coating in a combined single-step deposition process.

According to a first aspect of the present invention there is provided a method of surface treatment of at least part of an electro-conductive surface of an implant according to claim 1. Aptly, the method comprises the steps of: preparing an electrolyte solution containing calcium and phosphorus ions and a therapeutic agent; and electrochemically depositing a calcium phosphate coating incorporating said therapeutic agent on said electro-conductive surface of the implant.

In a preferred embodiment, the electrolyte solution contains in combination with the therapeutic agent a complexing agent such that the resulting complex has a net positive charge.

In the present invention, incorporation of the therapeutic agent into the calcium phosphate coating takes place during the electrochemical deposition of the coating. As the calcium phosphate coating and the therapeutic agent are deposited simultaneously the number of steps required in the manufacture of an implant is reduced. In contrast to conventional manufacturing processes, the present invention reduces the number of steps required to produce a suitable coating on the implant to a single step process. It will be appreciated, however, that multiple coating steps may be used to create tailored and time-dependent release of therapeutic agents.

Preferably, the therapeutic agent comprises an antimicrobial agent. Such an antimicrobial agent may comprise a metallic, an inorganic, organic or biological antimicrobial agent. Advantageously, the antimicrobial agent comprises a metal ion, in particular silver, copper, zinc or cobalt ions or a mixture of same.

In general, metal phosphates exhibit a low solubility in water at neutral to alkaline pH values. This is especially true for metals which are of interest for as antimicrobial agents, such as copper, silver and cobalt. If minute amount of these metals, as ions in the form of a metal salt, are added to a calcium phosphate solution an immediate precipitation reaction can be observed and the meant-to-be antimicrobial agent may be no longer available for deposition on an implant surface via electrochemical deposition. The use of a complexing agent overcomes this problem by keeping the metal ions in solution, for example as a positively charged ammine-complex. In addition to stabilization of the solution, this kind of complex has the advantage of being positively charged and hence is still electrostatically attracted by a negatively polarized cathode formed by the electro-conductive surface of the implant.

Preferably also, therefore, the complexing agent comprises an (di)-ammine complex building agent. Other complex forming molecules may, however, be used provided that the resulting complex has a net positive charge.

Calcium phosphates tend to precipitate in alkaline solutions whilst, for example, a silver diammin complex starts to dissociate at pH values below 6.

Preferably, therefore, during preparation of the electrolyte solution a base solution is formed prior to addition of the complexing agent that has a pH value between 7 and 9 inclusive.

As the invention does not necessarily involve any thermal post-treatment of the implant and takes place in a very moderate chemical and electrochemical environment, it is possible to include further actives such as organic or biologic, for example peptides, DNA, etc., molecules in the electrolyte solution provided that they produce the prerequisite of a positive charge in solution.

According to a second aspect of the present invention there is provided an implant according to claim 19. Aptly the implant has an at least partial electro-conductive surface which has undergone a surface treatment of at least part of said electro-conductive surface of an implant, the surface treatment comprising the steps of: preparing an electrolyte solution containing calcium and phosphorus ions and a therapeutic agent; and electrochemically depositing a calcium phosphate coating incorporating said therapeutic agent on said electro-conductive surface of the implant.

In a preferred embodiment, the electrolyte solution contains in combination with the therapeutic agent a complexing agent such that the resulting complex has a net positive charge.

Preferably, the implant has an electro-conductive surface formed by a titanium alloy.

Preferably also, the titanium alloy comprises Ti -6Al-7Nb, Ti-6Al-4V or commercially pure Ti.

In an alternative embodiment the implant has an electro-conductive surface formed by a zirconium alloy, in particular Zr-2.5Nb.

According to a third aspect of the present invention there is provided an electrolyte solution for use in the surface treatment of at least part of an electro-conductive surface of an implant comprising calcium and phosphorus ions and a therapeutic agent according to claim 24.

In a preferred embodiment a therapeutic agent is used in combination with a complexing agent such that the resulting complex has a net positive charge.

Further preferred but non-essential features of the various aspects of the present invention are described in the dependent claims appended hereto.

The various aspects of the present invention will now be further described by way of example.

In order to simultaneously, electrochemically deposit a therapeutic agent such as silver ions and a calcium phosphate coating in a combined single-step deposition process it is first necessary to prepare an electrolyte solution containing silver, calcium and phosphate ions. This can be achieved as follows.

First, a 1M ammonia solution is prepared to form the base electrolyte. The initial pH value of this solution is approximately 11 to 12 and must be reduced by adding 10M nitric acid until it is approximately 7.8 for the reasons indicated above. A predetermined amount of silver nitrate (AgN0₃) is then added. This amount may comprise, for example, 0.02g/l but up to ten times this quantity is possible if required, i.e. the predetermined amount may be between 0.01 g/l and 0.20 g/l inclusive. As indicated above, other metal salts can be used instead of or in addition to silver nitrate dependent on the metal ions it is desired to deposit on the implant.

After complete dissolution of the metal salt, ammonium phosphate and calcium nitrate are added to the electrolyte solution. Preferably, the Ca/P-ratio is 1.0. - 2.0, more preferably 1.2 - 1.8 and even more preferably 1.67 and the concentrations of calcium and phosphate are Calcium: 0.042 M and Phosphate: 0.025 M. The resulting electrolyte solution has a pH value before the start of the electrochemical deposition between 5.5 and 4.5 inclusive and stabilizes at 4.5.

Surface treatment of the implant can now take place using cathodic deposition to deposit a silver-containing calcium phosphate layer on the implant surface. Preferably, the implant or at least an electro-conductive surface of same comprises a titanium alloy, for example Ti -6Al-7Nb, which has been slightly etched in HF/HNO₃-solution prior to the electrochemical coating procedure. Alternatively, the implant or at least an electro-conductive surface of same comprises a titanium alloy in form of Ti-6Al-4V or commercially pure Ti. In a further alternative embodiment, the implant or at least an electro-conductive surface of same comprises a zirconium alloy, in particular in form of Zr-2.5Nb.

In one embodiment of the invention, during the electrochemical coating procedure the voltage used is between 1 and 3 V inclusive in potentiostatic mode. In another embodiment of the invention, during the electrochemical coating procedure the electric current used is between 0.001 and 0.2 mA/cm² inclusive in galvanostatic mode. Particularly preferred is an electric current of approximately 0.01 mA/cm². A graphite or titanium anode is also used and the electrolyte solution is constantly stirred using a magnetic stirrer. A typical process duration is 30 minutes but this is dependent on the desired coating thickness and structure. During the deposition process the pH of the solution changes to slightly higher values.

After treatment, the silver contained in the coating on the implant is at least partly of a metallic nature. Moreover, the base colour of the coating is grey to black dependent on the silver concentration used in the electrolyte solution.

As indicated previously, other or additional metal ions can be used as therapeutic agents, for example ions of zinc, copper, cobalt, aluminium can be used. Likewise, other complexing agents can be used to stabilize these metal ions in the electrolyte solution, for example an ethylenediamine complex.

In addition to metal ions, other antimicrobial actives such as antibiotics, antiviral drugs or fungicides may be added in an appropriate form to the electrolyte solution provided that these agents are positively charged in the electrolyte solution. Similarly, other therapeutic agents such as growth factors, bisphosphonates, peptides, DNA etc., can be used. It will thus be appreciated that an implant can be treated to produce multi-layer systems creating tailored release profiles of a variety of actives. Also, after treatment according to the invention additional actives can be incorporated into the already deposited layer using electrophoresis or similar techniques. To achieve this, the deposited calcium phosphate layer may be heat treated prior to the deposition of additional actives. A typical heat treatment is performed in a vacuum or inert gas furnace at 550°C/lh with appropriate heating and cooling rates.

It will be appreciated that the method of treatment of an implant in accordance with the present invention has several advantages over the prior art. First, the incorporation of a therapeutic agent in a calcium phosphate coating is possible using a simple single step coating process. No ion exchange reaction is needed and common chemicals can be used as electrolyte constituents. It is believed that in comparison to sol/gel coatings, the method of the present invention does not require any subsequent heat treatment to finalize the coating properties.

## Claims

1. A method of surface treatment of at least part of an electro-conductive surface of an implant comprising the steps of:
preparing an electrolyte solution containing calcium and phosphorus ions and a therapeutic agent, wherein the electrolyte solution comprises in combination with a therapeutic agent a complexing agent such that the resulting complex has a net positive charge; and
electrochemically depositing a calcium phosphate coating incorporating said therapeutic agent on said electro-conductive surface of the implant, wherein the therapeutic agent comprises metal ions and further wherein the complexing agent comprises a (di-) ammine complex building agent.

2. A method as claimed in Claim 1, wherein the therapeutic agent comprises an antimicrobial agent.

3. A method as claimed in Claim 1, wherein the therapeutic agent comprises silver, copper, zinc or cobalt ions or a mixture of same.

4. A method as claimed in any preceding claim, wherein during preparation of the electrolyte solution a base solution is formed prior to addition of the complexing agent that has a pH value between 7 and 9 inclusive.

5. A method as claimed in any preceding claim, wherein the preparation of the electrolyte solution comprises the following steps: preparing an ammonia solution to form a base electrolyte;
reducing the pH value of the ammonia solution to 8 or less by the addition of an acid thereto;
adding a predetermined quantity of a metal salt thereto; and
adding ammonium phosphate and calcium nitrate thereto after dissolution of the metal salt.

6. A method as claimed in Claim 5, wherein the metal salt comprises a silver salt.

7. A method as claimed in Claim 5 or Claim 6, wherein the metal salt is silver nitrate.

8. A method as claimed in any of Claims 5 to 7, wherein the predetermined quantity of the metal salt is between 0.01 g/l and 0.20 g/l inclusive.

9. A method as claimed in any of Claims 5 to 8, wherein the pH value of the ammonia solution is reduced by the addition of a mineral or organic acid thereto.

10. A method as claimed in Claim 9, wherein the pH value of the ammonia solution is reduced by the addition of nitric acid, hydrochloric acid, phosphoric acid, and/or acetic acid thereto.

11. A method as claimed in any of Claims 5 to 10, wherein the Ca/P-ratio in the electrolyte solution is 1,0 - 2.0, preferably 1.2. -1.8.

12. A method as claimed in any of Claims 5 to 11, wherein the pH value of the electrolyte solution is between 7.5 and 4.5 inclusive,

13. A method as claimed in any preceding claim, wherein during the electrochemical deposition of the calcium phosphate coating a voltage between 1 V and 3 V inclusive in potentiostatic mode is used.

14. A method as claimed in any preceding claim, wherein during the electrochemical deposition of the calcium phosphate coating an electric current between 0.001 and 0.2 mA/cm² inclusive in galbvanostatic mode is used.

15. A method as claimed in any preceding claim, wherein during the electrochemical deposition of the calcium phosphate coating the electrolyte solution is stirred using a magnetic stirrer.

16. A method as claimed in any preceding claim, wherein the electrochemical deposition process has a duration of the order of 10 - -120 minutes, preferably 15 - 60 minutes and more preferably 20 - 45 minutes.

17. A method as claimed in any preceding claim, wherein the complexing agent comprises an ethylenediamine.

18. A method of manufacturing an implant comprising the method of any one of the preceding claims.

19. An implant with an at least partial electro-conductive surface which has undergone a surface treatment of at least part of said electro-conductive surface of an implant, the surface treatment comprising the steps of:
preparing an electrolyte solution containing calcium and phosphorus ions and a therapeutic agent, in combination with a complexing agent such that the resulting complex has a net positive charge; and
electrochemically depositing a calcium phosphate coating incorporating said therapeutic agent on said electro-conductive surface of the implant, wherein the therapeutic agent comprises metal ions and further wherein the complexing agent comprises a (di-) ammine complex building agent.

20. An implant as claimed in Claim 19, wherein said electro-conductive surface is formed by a titanium alloy.

21. An implant as claimed in Claim 20. wherein said titanium alloy comprises Ti -6Al-7Nb, commercially pure Ti, and/or Ti6Al4V.

22. An implant as claimed in claim 19, wherein said electro-conductive surface is formed by a zirconium alloy, in particular Zr-2.5 Nb.

23. An implant as claimed in any of Claims 19 to 22, comprising an orthopaedic or a dental implant.

24. An electrolyte solution for use in the surface treatment of at least part of an electro-conductive surface of an implant comprising calcium, and phosphorus ions and a therapeutic agent, and comprising in combination with the therapeutic agent a complexing agent such that the resulting complex has a net positive charge, wherein the therapeutic agent comprises metal ions and further wherein the complexing agent comprises a (di-)ammine complex building agent

25. A solution as claimed in Claim 24, wherein the therapeutic agent comprises an antimicrobial agent.

26. A solution as claimed in Claim 25, wherein the therapeutic agent comprises silver, copper, zinc or cobalt ions or a mixture of same.

27. A solution as claimed in any of Claims 24 to 26, which comprises an ammonia solution.

28. A solution as claimed in any of Claims 24 to 27, produced by the following steps:
preparing an ammonia solution to form a base electrolyte;
reducing the pH value of the ammonia solution to less than 9 by the addition of an acid thereto;
adding a predetermined quantity of a metal salt thereto; and
adding a soluble phosphate and a calcium salt thereto after dissolution of the metal salt.

29. A solution as claimed in any of Claims 24 to 28, wherein the metal salt comprises a silver salt.

30. A solution as claimed in any of Claims 24 to 29 wherein the metal salt is silver nitrate.

31. A solution as claimed in any of Claims 24 to 30, wherein the predetermined quantity of the metal salt is between 0.01 g/l and 0,20 g/l inclusive.

32. A solution as claimed in any of Claims 29 to 31 wherein the acid is nitric acid.

33. A solution as claimed in any of Claims 24 to 32, wherein the Ca/P-ratio is 1.0-2.0, preferably 1.2-1.8.

34. A solution as claimed in any of Claims 24 to 33, which has a pH value between 7.5 and 4.5 inclusive.

## Patentansprüche

1. Ein Verfahren zur Oberflächenbehandlung zumindest eines Teils einer elektrisch leitenden Oberfläche eines Implantats, das die folgenden Schritte beinhaltet:
Zubereiten einer Elektrolytlösung, die Calcium- und Phosphorionen und ein Therapeutikum beinhaltet, wobei die Elektrolytlösung in Kombination mit einem Therapeutikum einen Komplexbildner beinhaltet, so dass der resultierende Komplex eine positive Nettoladung aufweist; und
elektrochemisches Abscheiden einer Calciumphosphatbeschichtung, welche das Therapeutikum inkorporiert, auf der elektrisch leitenden Oberfläche des Implantats, wobei das Therapeutikum Metallionen beinhaltet und wobei ferner der Komplexbildner ein (Di)amminkomplexherstellungsmittel beinhaltet.

2. Verfahren gemäß Anspruch 1, wobei das Therapeutikum ein Antimikrobiotikum beinhaltet.

3. Verfahren gemäß Anspruch 1, wobei das Therapeutikum Silber-, Kupfer-, Zink- oder Cobaltionen oder eine Mischung derselben beinhaltet.

4. Verfahren gemäß einem der vorhergehenden Ansprüche, wobei während der Zubereitung der Elektrolytlösung vor der Zugabe des Komplexbildners eine Basenlösung gebildet wird, die einen pH-Wert zwischen einschließlich 7 und 9 aufweist.

5. Verfahren gemäß einem der vorhergehenden Ansprüche, wobei die Zubereitung der Elektrolytlösung die folgenden Schritte beinhaltet: Zubereiten einer Ammoniaklösung zum Bilden eines Basen-Elektrolyten;
Verringern des pH-Werts der Ammoniaklösung auf 8 oder weniger durch die Zugabe einer Säure dazu;
Zugeben einer vorbestimmten Menge eines Metallsalzes dazu; und
nach der Auflösung des Metallsalzes, Zugeben von Ammoniumphosphat und Calciumnitrat dazu.

6. Verfahren gemäß Anspruch 5, wobei das Metallsalz ein Silbersalz beinhaltet.

7. Verfahren gemäß Anspruch 5 oder Anspruch 6, wobei das Metallsalz Silbernitrat ist.

8. Verfahren gemäß einem der Ansprüche 5 bis 7, wobei die vorbestimmte Menge des Metallsalzes zwischen einschließlich 0,01 g/l und 0,20 g/l beträgt.

9. Verfahren gemäß einem der Ansprüche 5 bis 8, wobei der pH-Wert der Ammoniaklösung durch die Zugabe einer Mineralsäure oder einer organischen Säure dazu verringert wird.

10. Verfahren gemäß Anspruch 9, wobei der pH-Wert der Ammoniaklösung durch die Zugabe von Salpetersäure, Salzsäure, Phosphorsäure und/oder Essigsäure dazu verringert wird.

11. Verfahren gemäß einem der Ansprüche 5 bis 10, wobei das Ca/P-Verhältnis in der Elektrolytlösung 1,0-2,0, vorzugsweise 1,2-1,8 beträgt.

12. Verfahren gemäß einem der Ansprüche 5 bis 11, wobei der pH-Wert der Elektrolytlösung zwischen einschließlich 7,5 und 4,5 beträgt.

13. Verfahren gemäß einem der vorhergehenden Ansprüche, wobei während der elektrochemischen Abscheidung der Calciumphosphatbeschichtung eine Spannung zwischen einschließlich 1 V und 3 V im potentiostatischen Modus verwendet wird.

14. Verfahren gemäß einem der vorhergehenden Ansprüche, wobei während der elektrochemischen Abscheidung der Calciumphosphatbeschichtung ein elektrischer Strom zwischen einschließlich 0,001 und 0,2 mA/cm² im galvanostatischen Modus verwendet wird.

15. Verfahren gemäß einem der vorhergehenden Ansprüche, wobei während der elektrochemischen Abscheidung der Calciumphosphatbeschichtung die Elektrolytlösung unter Verwendung eines Magnetrührers gerührt wird.

16. Verfahren gemäß einem der vorhergehenden Ansprüche, wobei der elektrochemische Abscheidungsprozess über eine Zeitdauer im Bereich von 10-120 Minuten, vorzugsweise 15-60 Minuten und noch bevorzugter 20-45 Minuten durchgeführt wird.

17. Verfahren gemäß einem der vorhergehenden Ansprüche, wobei der Komplexbildner ein Ethylendiamin beinhaltet.

18. Ein Verfahren zum Fertigen eines Implantats, das das Verfahren gemäß einem der vorhergehenden Ansprüche beinhaltet.

19. Ein Implantat mit einer zumindest teilweise elektrisch leitenden Oberfläche, das eine Oberflächenbehandlung zumindest eines Teils der elektrisch leitenden Oberfläche eines Implantats erfahren hat, wobei die Oberflächenbehandlung die folgenden Schritte beinhaltet:
Zubereiten einer Elektrolytlösung, die Calcium- und Phosphorionen und ein Therapeutikum beinhaltet, in Kombination mit einem Komplexbildner, so dass der resultierende Komplex eine positive Nettoladung aufweist; und
elektrochemisches Abscheiden einer Calciumphosphatbeschichtung, welche das Therapeutikum inkorporiert, auf der elektrisch leitenden Oberfläche des Implantats, wobei das Therapeutikum Metallionen beinhaltet und wobei ferner der Komplexbildner ein (Di)amminkomplexherstellungsmittel beinhaltet.

20. Implantat gemäß Anspruch 19, wobei die elektrisch leitende Oberfläche durch eine Titanlegierung gebildet wird.

21. Implantat gemäß Anspruch 20, wobei die Titanlegierung Ti-6Al-7Nb, technisch reines Ti und/oder Ti6Al4V beinhaltet.

22. Implantat gemäß Anspruch 19, wobei die elektrisch leitende Oberfläche durch eine Zirconiumlegierung gebildet wird, insbesondere Zr-2,5Nb.

23. Implantat gemäß einem der Ansprüche 19 bis 22, beinhaltend ein orthopädisches Implantat oder ein Zahnimplantat.

24. Eine Elektrolytlösung zur Verwendung bei der Oberflächenbehandlung zumindest eines Teils einer elektrisch leitenden Oberfläche eines Implantats, die Calcium- und Phosphorionen und ein Therapeutikum beinhaltet und in Kombination mit dem Therapeutikum einen Komplexbildner beinhaltet, so dass der resultierende Komplex eine positive Nettoladung aufweist, wobei das Therapeutikum Metallionen beinhaltet und wobei ferner der Komplexbildner ein (Di)amminkomplexherstellungsmittel beinhaltet.

25. Lösung gemäß Anspruch 24, wobei das Therapeutikum ein Antimikrobiotikum beinhaltet.

26. Lösung gemäß Anspruch 25, wobei das Therapeutikum Silber-, Kupfer-, Zink- oder Cobaltionen oder eine Mischung derselben beinhaltet.

27. Lösung gemäß einem der Ansprüche 24 bis 26, die eine Ammoniaklösung beinhaltet.

28. Lösung gemäß einem der Ansprüche 24 bis 27, die durch die folgenden Schritte erzeugt wird:
Zubereiten einer Ammoniaklösung zum Bilden eines Basen-Elektrolyten;
Verringern des pH-Werts der Ammoniaklösung auf weniger als 9 durch die Zugabe einer Säure dazu;
Zugeben einer vorbestimmten Menge eines Metallsalzes dazu; und
nach der Auflösung des Metallsalzes, Zugeben eines löslichen Phosphats und eines Calciumsalzes dazu.

29. Lösung gemäß einem der Ansprüche 24 bis 28, wobei das Metallsalz ein Silbersalz beinhaltet.

30. Lösung gemäß einem der Ansprüche 24 bis 29, wobei das Metallsalz Silbernitrat ist.

31. Lösung gemäß einem der Ansprüche 24 bis 30, wobei die vorbestimmte Menge des Metallsalzes zwischen einschließlich 0,01 g/l und 0,20 g/l beträgt.

32. Lösung gemäß einem der Ansprüche 29 bis 31, wobei die Säure Salpetersäure ist.

33. Lösung gemäß einem der Ansprüche 24 bis 32, wobei das Ca/P-Verhältnis 1,0-2,0, vorzugsweise 1,2-1,8 beträgt.

34. Lösung gemäß einem der Ansprüche 24 bis 33, die einen pH-Wert zwischen einschließlich 7,5 und 4,5 aufweist.

## Revendications

1. Une méthode de traitement de surface d'au moins une partie d'une surface électro-conductrice d'un implant comprenant les étapes de :
préparation d'une solution électrolytique contenant des ions calcium et phosphore et un agent thérapeutique, dans laquelle la solution électrolytique comprend en combinaison avec un agent thérapeutique un agent complexant, de telle sorte que le complexe résultant a une charge positive nette ; et
dépôt électrochimique d'un revêtement de phosphate de calcium incorporant ledit agent thérapeutique sur ladite surface électro-conductrice de l'implant, dans laquelle l'agent thérapeutique comprend des ions métalliques et dans laquelle en sus l'agent complexant comprend un agent de construction de complexe (di-) amine.

2. Une méthode telle que revendiquée dans la revendication 1, dans laquelle l'agent thérapeutique comprend un agent antimicrobien.

3. Une méthode telle que revendiquée dans la revendication 1, dans laquelle l'agent thérapeutique comprend des ions argent, cuivre, zinc ou cobalt ou un mélange de ceux-ci.

4. Une méthode telle que revendiquée dans n'importe quelle revendication précédente, dans laquelle durant la préparation de la solution électrolytique, une solution de base est formée préalablement à l'ajout de l'agent complexant qui a une valeur pH comprise entre 7 et 9 inclus.

5. Une méthode telle que revendiquée dans n'importe quelle revendication précédente, dans laquelle la préparation de la solution électrolytique comprend les étapes suivantes :
préparation d'une solution ammoniacale pour former un électrolyte de base ;
réduction de la valeur pH de la solution ammoniacale à 8 ou moins en ajoutant un acide à celle-ci ;
ajout d'une quantité prédéterminée d'un sel métallique à celle-ci ; et
ajout de phosphate d'ammonium et de nitrate de calcium à celle-ci après dissolution du sel métallique.

6. Une méthode telle que revendiquée dans la revendication 5, dans laquelle le sel métallique comprend un sel d'argent.

7. Une méthode telle que revendiquée dans la revendication 5 ou la revendication 6, dans laquelle le sel métallique est du nitrate d'argent.

8. Une méthode telle que revendiquée dans n'importe lesquelles des revendications 5 à 7, dans laquelle la quantité prédéterminée du sel métallique est comprise entre 0,01 g/l et 0,20 g/l inclus.

9. Une méthode telle que revendiquée dans n'importe lesquelles des revendications 5 à 8, dans laquelle la valeur pH de la solution ammoniacale est réduite en ajoutant un acide minéral ou organique à celle-ci.

10. Une méthode telle que revendiquée dans la revendication 9, dans laquelle la valeur pH de la solution ammoniacale est réduite en ajoutant de l'acide nitrique, de l'acide chlorhydrique, de l'acide phosphorique, et/ou de l'acide acétique à celle-ci.

11. Une méthode telle que revendiquée dans n'importe lesquelles des revendications 5 à 10, dans laquelle le rapport Ca/P dans la solution électrolytique est de 1,0 - 2,0, préférablement de 1,2 - 1,8.

12. Une méthode telle que revendiquée dans n'importe lesquelles des revendications 5 à 11, dans laquelle la valeur pH de la solution électrolytique est comprise entre 7,5 et 4,5 inclus.

13. Une méthode telle que revendiquée dans n'importe quelle revendication précédente, dans laquelle, durant le dépôt électrochimique du revêtement de phosphate de calcium, une tension comprise entre 1 V et 3 V inclus est utilisée en mode potentiostatique.

14. Une méthode telle que revendiquée dans n'importe quelle revendication précédente, dans laquelle, durant le dépôt électrochimique du revêtement de phosphate de calcium, un courant électrique compris entre 0,001 et 0,2 mA/cm² inclus est utilisé en mode galvanostatique.

15. Une méthode telle que revendiquée dans n'importe quelle revendication précédente, dans laquelle, durant le dépôt électrochimique du revêtement de phosphate de calcium, la solution électrolytique est agitée à l'aide d'un agitateur magnétique.

16. Une méthode telle que revendiquée dans n'importe quelle revendication précédente, dans laquelle le procédé de dépôt électrochimique a une durée de l'ordre de 10 - 120 minutes, préférablement de 15 - 60 minutes et plus préférablement de 20 - 45 minutes.

17. Une méthode telle que revendiquée dans n'importe quelle revendication précédente, dans laquelle l'agent complexant comprend une éthylènediamine.

18. Une méthode de fabrication d'un implant comprenant la méthode de l'une quelconque des revendications précédentes.

19. Un implant possédant une surface électro-conductrice au moins partielle, au moins une partie de ladite surface électro-conductrice d'un implant ayant subi un traitement de surface, le traitement de surface comprenant les étapes de :
préparation d'une solution électrolytique contenant des ions calcium et phosphore et un agent thérapeutique, en combinaison avec un agent complexant, de telle sorte que le complexe résultant a une charge positive nette ; et
dépôt électrochimique d'un revêtement de phosphate de calcium incorporant ledit agent thérapeutique sur ladite surface électro-conductrice de l'implant, dans lequel l'agent thérapeutique comprend des ions métalliques et dans lequel en sus l'agent complexant comprend un agent de construction de complexe (di-) amine.

20. Un implant tel que revendiqué dans la revendication 19, dans lequel ladite surface électro-conductrice est formée par un alliage de titane.

21. Un implant tel que revendiqué dans la revendication 20, dans lequel ledit alliage de titane comprend le Ti-6AI-7Nb, le Ti commercialement pur, et/ou le Ti6Al4V.

22. Un implant tel que revendiqué dans la revendication 19, dans lequel ladite surface électro-conductrice est formée par un alliage de zircone, en particulier le Zr-2,5 Nb.

23. Un implant tel que revendiqué dans n'importe lesquelles des revendications 19 à 22, comprenant un implant orthopédique ou dentaire.

24. Une solution électrolytique pour une utilisation dans le traitement de surface d'au moins une partie d'une surface électro-conductrice d'un implant comprenant des ions calcium et phosphore et un agent thérapeutique, et comprenant en combinaison avec l'agent thérapeutique un agent complexant, de telle sorte que le complexe résultant a une charge positive nette, dans laquelle l'agent thérapeutique comprend des ions métalliques et dans laquelle en sus l'agent complexant comprend un agent de construction de complexe (di-)amine.

25. Une solution telle que revendiquée dans la revendication 24, dans laquelle l'agent thérapeutique comprend un agent antimicrobien.

26. Une solution telle que revendiquée dans la revendication 25, dans laquelle l'agent thérapeutique comprend des ions argent, cuivre, zinc ou cobalt ou un mélange de ceux-ci.

27. Une solution telle que revendiquée dans n'importe lesquelles des revendications 24 à 26, laquelle comprend une solution ammoniacale.

28. Une solution telle que revendiquée dans n'importe lesquelles des revendications 24 à 27, produite par les étapes suivantes :
préparation d'une solution ammoniacale pour former un électrolyte de base ;
réduction de la valeur pH de la solution ammoniacale à moins de 9 en ajoutant un acide à celle-ci ;
ajout d'une quantité prédéterminée d'un sel métallique à celle-ci ; et
ajout d'un phosphate soluble et d'un sel de calcium à celle-ci après dissolution du sel métallique.

29. Une solution telle que revendiquée dans n'importe lesquelles des revendications 24 à 28, dans laquelle le sel métallique comprend un sel d'argent.

30. Une solution telle que revendiquée dans n'importe lesquelles des revendications 24 à 29, dans laquelle le sel métallique est du nitrate d'argent.

31. Une solution telle que revendiquée dans n'importe lesquelles des revendications 24 à 30, dans laquelle la quantité prédéterminée du sel métallique est comprise entre 0,01 g/l et 0,20 g/l inclus.

32. Une solution telle que revendiquée dans n'importe lesquelles des revendications 29 à 31, dans laquelle l'acide est de l'acide nitrique.

33. Une solution telle que revendiquée dans n'importe lesquelles des revendications 24 à 32, dans laquelle le rapport Ca/P est de 1,0 - 2,0, préférablement de 1,2 - 1,8.

34. Une solution telle que revendiquée dans n'importe lesquelles des revendications 24 à 33, laquelle a une valeur pH comprise entre 7,5 et 4,5 inclus.
